# EUROPÄISCHE PATENTANMELDUNG

(11) **EP 0 848 012 A1**
(43) Veröffentlichungstag der Anmeldung: **17.06.1998**
(21) Anmeldenummer: 96120024.3
(22) Anmeldetag: 13.12.1996
(51) Int. Cl.: C07K 14/52

(54) **Verwendung von Polypeptiden zur Behandlung von Thrombozytopenien**

(71) Anmelder: BOEHRINGER MANNHEIM GMBH, 68298 Mannheim (DE)
(72) Erfinder: Opitz, Hans-Georg, Dr., 69469 Weinheim (DE); Schmitt, Joachim, Dr., 68519 Viernheim (DE); Forssmann, Wolf-Georg, Prof.Dr., I.P.F., 30624 Hannover (DE); Schulz-Knappe, Peter, Dr., I.P.F., 30625 Hannover (DE)

(57) **Zusammenfassung**

Die Erfindung betrifft die Verwendung von Polypeptiden aus der Klasse der MIPs (macrophage inflammatory proteins) zur Behandlung von Erkrankungen, die mit einer pathologischen Veränderung der Blutplättchenbildung einhergehen, insbesondere zur Behandlung der Thrombozytopenie.

## Beschreibung

Die Erfindung betrifft die Verwendung von Polypeptiden aus der Klasse der MIPs (macrophage inflammatory proteins) zur Behandlung von Erkrankungen, die mit einer pathologischen Verminderung der Blutplättchenbildung einhergehen.

Thrombozyten spielen eine zentrale Rolle bei der Blutgerinnung und der Wundheilung. Thrombozytenerkrankungen können somit zu Störungen in der hämostatischen Blutstillung und damit zu massiven Blutungen führen. Man unterscheidet generell Thrombozytopenien, bei denen die Anzahl der Thrombozyten erniedrigt ist, und Thrombozytendysfunktionen, bei denen trotz normaler Thrombozytenzahlen deren Funktion gestört ist

Als Ursachen einer Thrombozytopenie kommen verminderte oder fehlende Megakaryozyten im Knochenmark, verminderte Plättchenproduktion, Sequestration der Thrombozyten in der Milz, vermehrter Thrombozytenabbau, vermehrter Thrombozytenverbrauch oder Verdünnung des Thrombozytenpools in Frage. Gleich welcher Ätiologie, führt eine schwere Thrombozytopenie zu Hautblutungen in Form von multiplen Petechien, die meist an den Unterschenkeln am deutlichsten hervortreten, sowie zu verstreuten kleinen Ekchymosen nach Bagatelltraumen. Gravierender sind jedoch Schleimhautblutungen (Nasenbluten; Blutungen im Gastrointestinaltrakt, im Urogenitaltrakt und in der Vagina) sowie Blutungen nach chirurgischen Eingriffen. Abhängig vom Ausmaß der Thrombozytopenie können schwere gastrointestinale Blutungen sowie Blutungen in das Zentralnervensystem mit lebensbedrohlichen Folgen auftreten.

Thrombozytopenien können bei einer Vielzahl von Erkarankungen und bei bestimmten therapeutischen Eingriffen beobachtet werden Eine verminderte Plättchenbildung kann auftreten in Folge, z.B. einer myelosuppressiven Therapie (Bestrahlung, Chemotherapie insbesonder Hochdosistherapie), einer Knochenmarktransplantation, einer Leukämie, einer Anämie (aplastische Anämie, Fanconi Anämie etc.) oder auch nach Alkoholabusus. Einem vermehrten Thrombozytenverbrauch oder -abbau können Erkrankungen zugrunde liegen, wie Idiopathische Thrombozytopenische Purpura, Infektionen Thrombozytopenie bei HIV-Infektion, Sepsis etc.), immunologische Grunderkrankungen Autoimmunerkrankungen wie z.B Systemischer Lupus Erythematodes), chronische Leukämie oder maligne Lymphome. Eine verminderte Plättchenzahl kann auch als medikamenteninduzierte Thrombozytopenie nach Gabe von beispielsweise Heparin, Chinidin, Sulfonamiden, orale Antidiabetika, Goldsalzen oder Rifampicin auftreten

Im Rahmen der Diagnostik in der klinischen Chemie ist die Bestimmung der Zellzahlen im peripheren Blutbild der wesentliche Parameter, um das Ausmaß der Thrombozytopenie festzustellen und erste Hinweise auf mögliche Ursachen zu erhalten. So weist z.B. ein vermehrter Anteil an großer Thrombozyten (bestimmt anhand des Blutausstriches oder durch Messung des mittleren Thrombozytenvolumens in einem elektronischen Blutzellanalysegerät) auf eine kompensatorisch erhöhte Thrombozytenproduktion hin. Diese findet man oft bei sekundären Thrombozytopenien aufgrund eines erhöhten Abbaus oder Verbrauchs der Thrombozyten. Die Blutungszeit ist bei schweren Thrombozytopenien jeglicher Ursache verlängert. Bei Patienten mit nur mäßiger Thrombozytopenie (z.B. 50000/ml Thrombozyten) kann die Bestimmung der Blutungszeit jedoch eine wertvolle Information sein. Eine stark verlängerte Blutungszeit weist in dem Fall darauf hin, daß. Beladung der Thrombozyten mit Antikörpern offensichtlich zu einer Funktionsstörung der zirkulierenden Thrombozyten führt. Ein weiteres diagnostisches Kriterium stellen die Untersuchungen von Knochemarkpunktionen dar. Die Thrombozyptopenien können dabei nach Anzahl und Erscheinungsbild der Megakaryozten beurteilt werden.

Zur Behandlung von Thrombozytopenien müssen die auslösenden Ursachen erkannt und korrigiert werden, z.B. bei medikamenten-induzierten Thromboytopenien durch rasches Absetzen des die Thrombozytopenie auslösenden Medikamentes, oder durch Erkennung und Behandlung einer Infektion mit endotoxinbildenden gramnegativen Keimen Wenn die Thrombozytopenie Folge einer Störung der Megakaryopoese und damit verminderten Produktion von Plättchen nach z.B einer Chemotherapie ist, kann durch Gabe von Thrombozytenkonzentraten die Thrombozytenzahl meist für die Dauer von 2-3 Tagen angehoben werden. Zur Behandlung der Thrombozytopenie werden Thrombozytenkonzentrate entweder kontinuierlich (1 bis 2 Einheiten pro Stunde) oder in größeren Mengen im Abstand von einigen Stunden gegeben, z.B. 6 bis 8 Einheiten alle 4 bis 6 Stunden. Prophylaktisch sollten Thrombozytenkonzentrate jedoch mit Zuräckhaltung eingesetzt werden, da wegen der Entwicklung von Thrombozyten-Alloantikörpern ihre Effektivität bei wiederholter Anwendung nachlassen kann. Auch darf bei Transfusionen das Risiko einer Infektion nicht vernachlässigt werden. Wenn eine rasche Regeneration der Knochenmarkfunktion nicht zu erwarten ist, sollte die Thrombozytentransfusion nur für die Behandlung einer klinisch manifesten Blutungsneigung eingesetzt werden. Bei Thrombozytopenien infolge vermehrten Plättchenverbrauchs sollten Thrombozytentransfusionen nur in Ausnahmefällen prophylaktisch gegeben werden, da bei diesen Erkrankungen die Thrombozyten innerhalb einer bis weniger Stunden wieder aus dem Kreislauf eliminiert werden Bei der Behandlung der heparininduzierten Thrombozytopenie können transfundierte Thrombozyten auch zur Bildung von Thrombozyten-Fibrin-Thromben und so zu einer schweren Thromboseneigung führen

Polypeptide aus der Klasse der MIPs (macrophage inflammatory proteins) sind bekannt aus WO 95/17092 Diese Polypeptide werden beispielsweise von Makrophagen oder Lymphozyten sezerniert, wenn diese durch gram-negative Bakterien, Lipopolysaccharide oder Concanavalin A stimuliert werden. WO 95/17092 beschreibt derartige Polypeptide und deren Verwendung zur Herstellung von Arzneimitteln zur Behandlung von Infektionen, Krebs, Entzundungen, myelopoetischen Dysfunktionen oder Autoimmunerkrankungen. Insbesondere wird in Zusammenhang mit der Hämatopoese die Hemmung von Knochenmarkstammzellen durch MIPs beschrieben, um beispielsweise myeloproliferative Erkrankungen zu behandeln. Die hemmende Wirkung von MIPs auf die Stammzellproliferation wird auch als Therapieprinzip in der Krebsbehandlung beschrieben, um Stammzellen durch vorhergehende Verabreichung von MIPs ruhigzustellen und so vor den Nebenwirkungen einer Chemotherapie zu schutzen. Daruberhinaus beschreibt WO 95/17092 Inhibitoren bzw. Antagonisten gegen derartige Polypeptide, die eine von MIPs verursachte Knochenmarkssuppression aufheben sollen und damit zur Behandlung von z.B. der aplastischen Anämie oder des myelodysplastischen Syndroms eingesetzt werden können. Eine Behandlung der Thrombozytose durch Gabe von MIPs soll durch Erhöhen der Gefäßpermeabilität in der Peripherie und damit vermehrter Sequestration von Thrombozyten erreicht werden. Für das aus WO 95/17092 bekannte Peptid MIP1γ ist bekannt, daß es die Reifung der Vorläuferzellen für Monozyten/Makrophagen in Gegenwart von MIP1γ inhibiert. MIP1γ wird demzufolge als M-CIF (Macrophage colony inhibition factor) bezeichnet.

Ferner ist aus DE 43 44 387 ein weiteres Peptid aus der Klasse der MIPs mit der Bezeichnung HCC-1 bekannt. Bei diesem Fragment handelt es sich uni ein Polypeptid, das die Sequenz von MIP-3 (1-69) umfaßt, und N-terminal um fünf weitere Aminosäuren verlängert ist. Dieses Peptid findet therapeutisch Verwendung zur Behandlung von Störungen der Migration von Zellen, Erkrankungen des Immunsystems, Tumoren sowie Dysfanktion regulatorischer Wachstumsfaktoren.

Überraschenderweise wurde nun gefunden, daß die Polypeptide aus der Klasse der MIPs und deren Derivate eine signifikante Erhöhung der Blutplättchenzahl bewirken und somit zur Herstellung von Arzneimitteln zur Behandlung von Zuständen, die mit einer Verringerung der Thrombozytenzahlen einhergehen, verwendet werden können. Dies umfaßt Erkrankungen, die entweder mit einer Störungen der Megakaryopoese oder einer pathologischen Verminderung der Blutplättchenbildung einhergehen. In diesem Zusammenhang werden diese Polypeptide insbesondere zur Behandlung von Thrombopenien verwendet, wie beispielsweise Thrombopenien, die durch Chemotherapie oder Bestrahlung ausgelöst wurden, oder Thrombopenien, die im Zusammenhang mit Knochenmarktransplantationen, viralen oder retroviralen Infektionen. Autoimmunerkrankungen und Anämien stehen. Die therapeutische Wirkung dieser Polypeptide wurde in vivo durch Stimulation der Thrombopoese nachgewiesen Dabei konnte nach einer vorhergehenden Behandlung mit Chemotherapeutika ein schnelleres Erholen der Vorläuferzellen im Knochenmark und damit eine deutlich verkürzte Dauer des thrombopenischen Zustandes erreicht werden.

Als Derivate der MIPs kommen auch solche Polypeptide in Frage, die durch Verlängerung, Deletion oder Substiution einzelner oder mehrer Aminosäuren abgeleitet werden. Derartige Fragmente oder Derivate sind beispielsweise solche Polypeptide, in denen eine oder mehrere Aminosauren durch natürliche oder andere Aminosäuren ersetzt werden. eine oder mehrere der Aminosäuren einen Substituenten aufweisen oder das Polypeptid an andere Verbindungen (wie z.B Polyethylenglykol) gekoppelt werden, um beispielsweise die Halbwertszeit zu erhöhen Ferner können die Polypeptide N- und/oder C-terminal um eine oder mehrere Aminosäuren verlängert oder verkürzt werden, wobei jedoch im wesentlichen die biologische Aktivität der Polypeptide im Hinblick auf die Stimulation der Thrombopoese erhalten bleibt Entsprechende Verlängerungen sind insbesondere Leadersequenzen oder sekretorische Sequenzen, wobei bis zu 40 Aminosäuren, bevorzugt bis zu 20 oder 10 Aminosäuren N-terminal der Sequenz des reifen Polypeptids vorausgehen können Fragmente sind solche Polypeptide, die sich durch N- oder C-terminale Verkürzungen der Sequenz des reifen Proteins ableiten, wobei die Verkürzungen 1 - 10 Aminsäuren umfassen können. Besonders bevorzugt in diesem Sinne kommt das Peptid HCC-1 in Frage.

Die biologische Wirksamkeit der Fragmente wird beispielhaft anhand von Untersuchungen des Polypeptids HCC-1 beschrieben.

### Beispiel

### Stimulation der Thrombopoese durch HCC-1 nach Busulfan-Behandlung von Balb/C Mäusen

### Material und Methoden:

Balb/C Mäusen (weiblich, 6-8 Wochen alt, 17 g. Körpergewicht) wird einmalig an Tag 0 Busulfan in einer Dosierung von 20 mg/kg intraperitoneal appliziert. Als Lösungsvermittler für Busulfan dient Cremophor EL. Beginnend mit Tag 1 bis Tag 15 wird den Tieren täglich einmal HCC-1 in PBS/1% BSA als Lösungsmittel intraperitoneal verabreicht. Die applizierten Dosen liegen zwischen 0.01 und 10 µg/Maus. Kontrolltiere erhalten nur Lösungsmittel PBS/1% BSA einmal täglich i.p.. Zu den angegebenen Zeitpunkten wird den Tieren 20 µl Vollblut retrobulbär entnommen und die verschiedenen Blutparameter in einem Blutanalysator (Contraves Autolyzer 801) bestimmt.

### Ergebnis:

Nach Application des Zytostatikums Busulfan wird in Mäusen bei geeigneter Dosierung eine vorübergehende Thrombozytopenie induziert (Morley und Blake, Oyekan und Onabanjo). Der Verlauf der Plättchenzahlen im peripheren Blut von Balb/C Mäusen nach Einmalgabe von Busulfan 20 mg/kg wurde über einen Zeitraum von 40 Tagen untersucht wobei an den Tagen 1-15 täglich eine Menge von 0.01 µg/Maus. 0.1 µg/Maus. 1 µg/Maus und 10 µg/Maus i.p. verabreicht wurde. Beginnend mit Tag 8 nach der Injektion fallen die Plättchenzahlen in der Busulfankontrolle ab und erreichen ihren Nadir bereits zwischen Tag 11 und 13 Die Reduktion der PLT-Werte betragt dabei etwa 60% Bis Tag 21 steigen die Plättchenzahlen dann wieder an, ein Reboundphänomen ist nicht zu beobachten Bis Tag 35 bleiben die PLT-Werte in der Busulfangruppe unterhalb der unbehandelten Kontrolle und erreichen das Ausgangsniveau nicht mehr erreicht.

Nach Busulfängabe und anschließender täglicher Application von HCC-1 kommt es ebenfalls ab Tag 8 zu einem Abfall der Plättchenzahlen Die erreichten Werte für den Nadir liegen jedoch bei allen Dosierungen von HCC-1 oberhalb der Busulfankontrolle Daruberhinaus wird der Zeitraum bis zum Wiederanstieg der Plättchenzahlen deutlich verkürzte so daß in der Gruppe mit HCC-1 1 µg/Maus bereits an Tag 15 wieder PLT-Werte um 800 x 10⁶/ml gemessen werden können. Im weiteren Verlauf des Experiments liegen die PLT-Zahlen in den HCC-1 behandelten Gruppen oberhalb der Busulfankontrolle und erreichen etwa ab Tag 30 wieder das Niveau von unbehandelten Kontrolltieren Legt man als Grenze 70 x 10⁶ PLT/ml fest, so wird die Dauer und das Ausmaß der Thrombopenie (gemessen als Fläche unter der Kurve) durch die Applikation von HCC-1 im Vergleich zur Busulfankontrolle uni maximal 70% reduziert.

**Tabelle 1**

| Einfluß von rmu TPO und HCC-1 auf das Ausmaß der Thrombopenie in Busulfan-behandelten Balb/C-Mäusen (Exp. 960800) | | | | |
|---|---|---|---|---|
| **Busulfan** | **Therapie** | **Dosis [pro Maus]** | **Thrombopenie**^{**a**} | |
| | | | **[%]** | **% Reduktion** |
| + | PBS-BSA | - | 100 | - |
| + | rmuTPO | 100 ng | n.d. | n.d. |
| + | HCC-1 | 10 µg | 45 | 55 |
| + | HCC-1 | 1 µg | 30 | 70 |
| + | HCC-1 | 0,1 µg | 35 | 65 |
| + | HCC-1 | 0,01 µg | 58 | 42 |

| | | | | |
|---|---|---|---|---|
| ^{a}= der Beginn der Thrombopenie wurde auf 700x10⁶ PLT/ml festgelegt und die Fläche unterhalb der Kurve für jede Therapiegruppe ermittelt (n=8 Tiere/Gruppe) | | | | |

## Patentansprüche

1. Verwendung von Polypeptiden aus der Klasse der MIPs (macrophage inflammatory peptides) zur Herstellung von Arzneimitteln zur Behandlung von Erkrankungen, die mit einer pathologischen Verminderung der Blutplättchenbildung einhergehen

2. Verwendung nach Anspruch 1 zur Behandlung von Erkrankungen, die mit einer Senkung der Megakaryopoese einhergehen.

3. Verwendung nach Anspruch 1 oder 2 zur Behandlung von Thrombopenien.

4. Verwendung nach Anspruch 3 zur Behandlung von Thrombopenien, die durch Chemotherapie oder Bestrahlung ausgelöst wurden.

5. Verwendung nach Anspruch 3 zur Behandlung von Thrombopenien, die im Zusammenhang mit Knochenmarktransplantationen, viralen oder retroviralen Infektionen Autoimmunerkrankungen und Anämien stehen.

6. Verwendung nach einem der Ansprüche 1-5, dadurch gekennzeichnet, daß das Polypeptid HCC-1 oder ein Derivat davon ist.

7. Verwendung nach einem der Ansprüche 1 - 5, dadurch gekennzeichnet, daß das Polypeptid MIP-1, MIP-3 oder MIP-4 oder ein Derivat hiervon ist.
